# EUROPÄISCHE PATENTANMELDUNG

(11) **EP 1 114 650 A2**
(43) Veröffentlichungstag der Anmeldung: **11.07.2001**
(21) Anmeldenummer: 01100360.5
(22) Anmeldetag: 05.01.2001
(51) Int. Cl.: A61M 5/00

(54) **Vorrichtung zum Erwärmen und/oder Warmhalten von medizinischen Lösungen in Notfallkoffern, Notfallrucksäcken u.dlg.**

(30) Priorität: 08.01.2000 DE 20000304 U
(71) Anmelder: Hofheinz, Andreas, 35715 Dietzhölztal (DE)
(72) Erfinder: Hofheinz, Andreas, 35715 Dietzhölztal (DE)
(74) Vertreter: Olbricht, Karl Heinrich, Dipl.-Phys.

(57) **Zusammenfassung**

Eine Vorrichtung (10) zum Erwärmen und/oder Warmhalten von Infusionslösungen in tragbaren Notfallbehältern (20), wie Notfallkoffern, -rucksäcken, -taschen u.dgl., besitzt wenigstens eine an eine externe Stromversorgung (30) anschließbare Heizeinrichtung (40) für Infusionsbehälter (12). Jede Heizeinrichtung (40) ist über Stromkontakte (50), beispielsweise Magnet-, Steck- oder Schleifkontakte, mit der externen Stromversorgung (30) verbindbar, wobei die Stromkontakte (30) frei zugänglich im Außenbereich des Notfallbehälters (20) angeordnet sind. Die Heizeinrichtung (40) ist bevorzugt eine in einem Aufnahmefach (22) des Notfallbehälters (20), in einer Wärmemanschette (42) oder in einer Wärmetasche integrierte Widerstandsheizung, deren Leistung mittels einer elektrischen oder elektronischen Steuerung (60) regelbar ist.

## Beschreibung

Die Erfindung betrifft eine Vorrichtung zum Erwärmen und/oder Warmhalten von medizinischen Lösungen, insbesondere Infusionslösungen, Transfusionslösungen u.dgl., gemäß Anspruch 1.

In der Notfallmedizin werden einem Patienten noch am Unfallort Infusionslösungen zugeführt, die als Trägerlösung für Medikamente oder zum Aufbau und zur Aufrechterhaltung des Kreislaufs dienen. Entsprechende Präparate werden gewöhnlich in Infusionsflaschen oder -beuteln bevorratet und direkt aus der Flasche bzw. dem Beutel über ein Zuleitungssystem in den Kreislauf infundiert. Gleiches gilt für Transfusionslösungen, welche im Bedarfsfalle noch am Unfallort dem menschlichen Körper zugeführt werden müssen.

Um während einer Infusion oder einer Transfusion eine Hypothermie des Patienten zu vermeiden, müssen die Lösungen auf eine für den menschlichen Organismus verträgliche Temperatur von etwa 28 bis 35°C gebracht werden. Hierzu verwendet man beispielsweise stationäre Thermoboxen, in der mehrere Infusionsflaschen oder -beutel auf Körpertemperatur bzw. gemäß Normvorgaben auf 37 °C erwärmt und auf dieser Temperatur gehalten werden. Kommt es zum Notfalleinsatz, müssen die Behälter aus der Thermobox entnommen und in einem Notfallkoffer oder -rucksack verstaut werden, was unter Umständen bereits wertvolle Zeit kosten kann.

Die unmittelbare Vorhaltung in Notfallkoffern oder Notfallrucksäcken kann diese Zeit sparen. Sie ist jedoch meist hyphotherm, d.h. je nach Dauer der Lagerung und/oder der Anfahrt kühlt sich die Infusionslösung bereits so weit ab, daß diese am eigentlichen Einsatzort keine ausreichende Temperatur mehr aufweist. Selbst in Fahr- oder Flugzeugen, die über ständig vorgeheizte Patientenräume verfügen oder die in beheizten Garagen stehen, ist die Temperatur der Infusionslösungen oft zu niedrig. Geringe Außentemperaturen können diese Situation dramatisch verschlechtern.

Neben Vorrichtungen zum vorwiegend stationären Temperieren von Infusionsflaschen oder -beuteln in Krankenhäusern oder Rettungsstationen (siehe beispielsweise DE-A1-43 28 321 oder DE-C1-195 03 350) hat man auch bereits mobil einsetzbare Geräte bzw. Vorrichtungen vorgeschlagen, die unmittelbar am Unfallort Anwendung finden sollen. So schlägt z.B. DE-U1-92 01 792 ein mobil einsetzbares Infusionserwärmungs- und Warmhaltegerät vor mit einer flexiblen Ummantelung aus wärmeisolierendem Material für die zu erwärmende Infusionsflasche sowie für das an der Flasche anzuschließende Zuleitungssystem mit Tropfkammer, Mengenregler und Schlauchleitung. In der mittels Klettverschlüssen zu verschließenden Ummantelung ist eine Widerstandsheizung in Form von Widerstandsdrähten integriert, deren Temperatur über eine elektrische Schaltung regelbar und anzeigbar ist. Für die Stromversorgung sind in einem Gerätegehäuse Akkumulatoren integriert, die zwischen den Einsätzen in einer geeigneten Ladestation aufgeladen werden.

Eine derartige Vorrichtung ist jedoch nur bedingt für den oft hektischen Alltag des Rettungsnotdienstes geeignet. Die elektrische Steuerung des Gerätes mit Anzeige und Stromversorgung hat neben den Infusionsflaschen einen relativ großen Platzbedarf, der in einem Notfallkoffer oder Notfallrucksack oft nicht vorhanden ist. Hinzu kommt, daß die Akkumulatoren eine nicht unerhebliche Gewichtsbelastung darstellen, was den mobilen Einsatz zusätzlich erschweren kann, insbesondere an Einsatzorten, die weitab von Rettungsfahrzeugen oder sonstigen quasi-stationären Einrichtungen liegen, beispielsweise in der alpinen Bergrettung, der Luftrettung oder der Seenotrettung. Darüber hinaus ist das Auswechseln einer Infusionsflasche äußerst zeitaufwendig. Die aluminiumbeschichtete Ummantelung kann nicht zuletzt durch die integrierten Widerstandsdrähte leicht beschädigt werden.

Es ist ein wichtiges Ziel der Erfindung diese und weitere Nachteile des Standes der Technik zu vermeiden und eine Vorrichtung zum Erwärmen und/oder Warmhalten von medizinischen Lösungen, insbesondere Infusionslösungen, Transfusionslösungen u.dgl., für den präklinischen Rettungsdienst zu schaffen, die eine Abkühlung der Infusions- bzw. Transfusionslösungen in Notfallkoffern, Notfallrucksäcken u.dgl. vor Gebrauch weitestgehend verhindert und einen unmittelbaren Einsatz ohne Zeitverzögerung ermöglicht. Die mit einfachen Mitteln kostengünstig herstellbare Vorrichtung soll ferner leicht zu handhaben und auch härteren Belastungen problemlos standhalten.

Hauptmerkmale der Erfindung sind in Anspruch 1, 20 und 21 angegeben. Ausgestaltungen sind Gegenstand der Ansprüche 2 bis 19.

Als Lösung sieht die Erfindung vor, daß bei einer Vorrichtung zum Erwärmen und/oder Warmhalten von medizinischen Lösungen, insbesondere Infusionslösungen, Transfusionslösungen u.dgl., in tragbaren Notfallbehältern, wie Notfallkoffern, -rucksäcken, -taschen u.dgl., mit wenigstens einer an eine externe Stromversorgung anschließbaren Heizeinrichtung für Infusionsbehälter und mit elektrischen Anschlüssen für die Heizeinrichtungen, die Anschlüsse derart im Außenbereich des Notfallbehälters angeordnet sind, daß durch Abstellen des Notfallbehälters in einem dafür vorgesehenen Bereich oder an einem dafür vorgesehenen Platz eine elektrische Verbindung zwischen der externen Stromversorgung und der bzw. den Heizvorrichtungen herstellbar ist.

Damit ist es möglich, einen vollständig bestückten Notfallbehälter an einem entsprechend eingerichteten, gut zugänglichen Ort in einem Rettungsfahrzeug oder -flugzeug abzustellen und im Einsatzfall rasch zu greifen. Durch die extern stromversorgte Heizeinrichtung ist sichergestellt, daß die Temperatur der Infusionslösung in dem jeweiligen Infusionsbehälter stets vor der Anwendung am Notfallort auf eine für den menschlichen Körper geeignete Temperatur vorgewärmt ist. Wird der Notfallbehälter nicht benötigt, wird die Temperatur gehalten.

Ein weiterer Vorteil besteht darin, daß der Notfallbehälter stets vollständig einsatzbereit ist. Eine nachträgliche oder ergänzende Bestückung mit Infusionsflaschen ist nicht notwendig, was im konkreten Einsatzfall wertvolle Zeit sparen kann. Der Forderung im modernen Rettungsdienst nach schneller und einfacher Handhabung von Notfallausrüstungen kann folglich durch die Erfindung Rechnung getragen werden.

Durch die weitere Ausgestaltung der Erfindung ist es möglich, bereits vorhandene Notfallkoffer, -rucksäcke, -taschen u.dgl. nachträglich mit einer Vorrichtung zum Erwärmen und/oder Warmhalten von medizinischen Lösungen kostengünstig auszustatten.

Geeignete Temperatur-Meß- und -Regel-Einrichtungen ermöglichen die Anpassung der Infusionstemperaturen an individuelle Bedürfnisse, beispielsweise unterschiedliche Anfahrts- oder Bergungswege.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- Fig. 1: eine schematische Darstellung einer Vorrichtung zum Erwärmen und/oder Warmhalten von Infusionslösungen,
- Fig. 2: eine Draufsicht auf die Vorrichtung von Fig. 1, teilweise im Schnitt,
- Fig. 3: einen Notfallkoffer mit einer Vorrichtung zum Erwärmen und/oder Warmhalten von Infusionslösungen,
- Fig. 4: einen Notfallrucksack mit einer Vorrichtung zum Erwärmen und/oder Warmhalten von Infusionslösungen und
- Fig. 5: eine Notfalltasche mit einer Vorrichtung zum Erwärmen und/oder Warmhalten von Infusionslösungen.

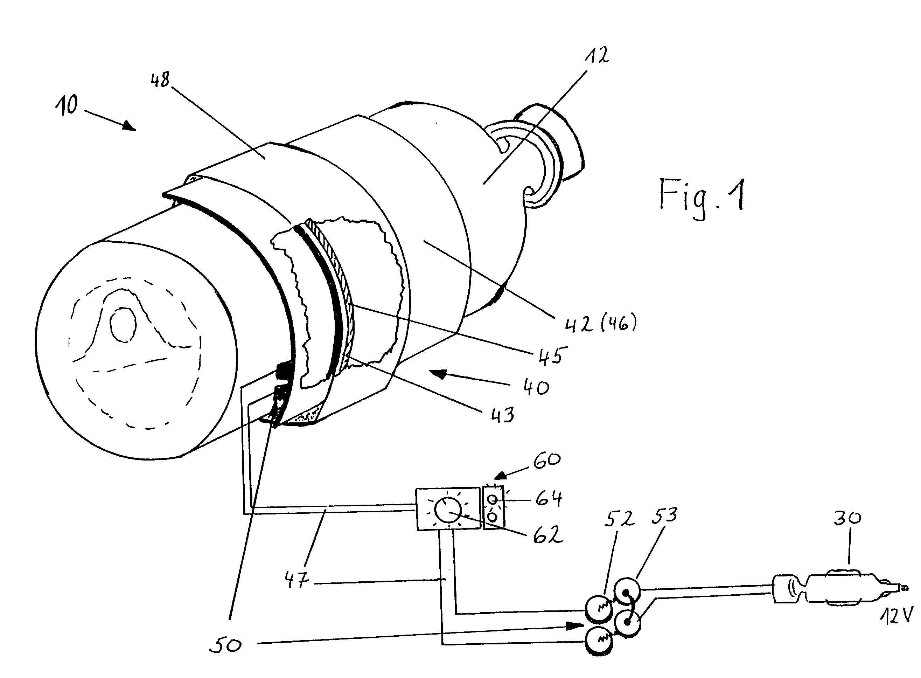

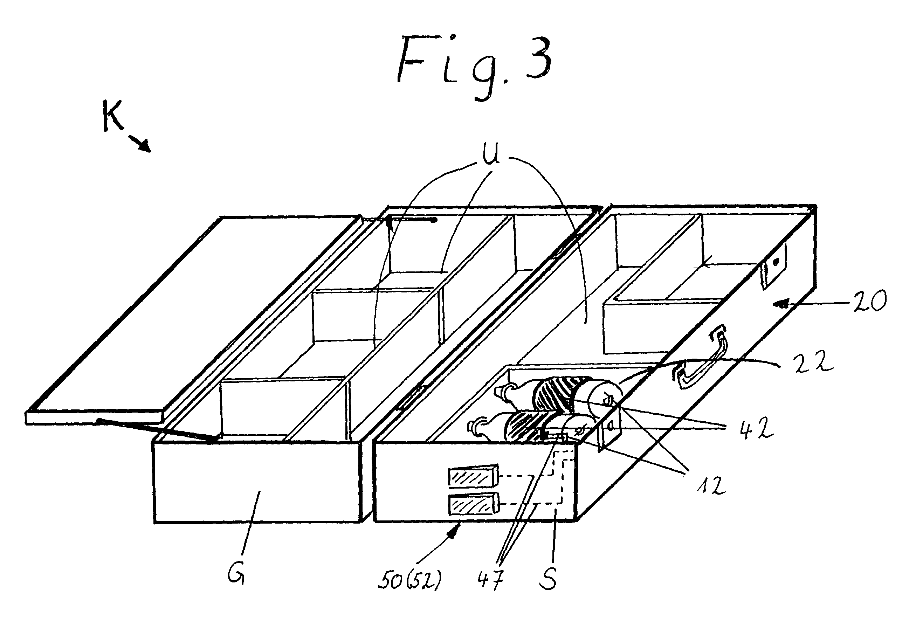

Die in Fig. 1 allgemein mit 10 bezeichnete Vorrichtung zum Erwärmen und/oder Warmhalten von Infusionslösungen ist für den Einsatz in einem tragbaren Notfallbehälter 20, beispielsweise einem Notfallkoffer, einem Notfallrucksack oder einer Notfalltasche ausgebildet. Sie umfaßt eine Heizeinrichtung 40 mit elektrischen Anschlüssen 50, die frei zugänglich im Außenbereich des Notfallbehälters 20 ausgebildet sind (siehe z.B. Fig. 3), um die Heizeinrichtung 40 mit einer außerhalb des Notfallbehälters 20 befindlichen Stromversorgung 30 zu verbinden.

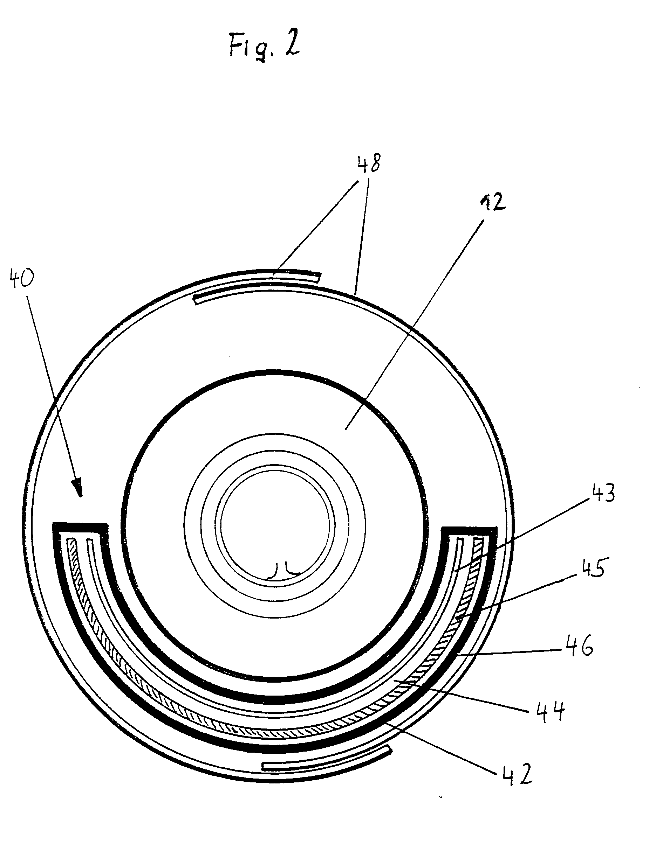

Die Heizeinrichtung 40 ist, wie insbesondere Fig. 2 zeigt, bevorzugt eine Widerstandsheizung in Form einer Wärmemanschette 42 oder -tasche, die zumindest abschnittsweise um eine Infusionsflasche 12 festlegbar ist. Die Manschette 42 hat in einer mechanisch belastbaren Ummantelung 46 eine Heizfolie 43 und eine auf dieser rückseitig aufgebrachte Wärmeisolierung 45 aus elastisch-formstabilem Material. Zur Befestigung der insgesamt flexiblen Wärmemanschette 42 verwendet man zweckmäßig Klettbänder 48 oder eine die Infusionsflasche 12 umschließende (nicht gezeigte) Gummimanschette, so daß stets eine rasche Entnahme der Infusionsflasche 12 möglich ist. Aufgrund der Wärmedurchleitung in Flüssigkeiten ist eine ständige jedoch nicht exakte Kontaktfläche zwischen der Wärmemanschette 42 und dem zu wärmenden Behälter 12 erforderlich, so daß unterschiedliche Volumina erwärmt werden können.

Jede Heizfolie 43 ist mit elektrischen Zuleitungen 47 versehen, die aus der Ummantelung 46 herausgeführt und mit den Anschlüssen 50 verbunden sind. Letztere sind Stromkontakte 52, 53 in Form von Magnetkontakten, Steckkontakten oder Schleifkontakten. Eine in der Zuleitung 47 vorgesehene elektrische Steuerung 60 hält mittels einem oder mehreren (nicht dargestellten) Temperaturfühlern die von der Wärmemanschette 42 erzeugte Temperatur auf konstantem Niveau, wobei die Temperaturfühler an einer oder mehreren Stellen der Manschette 42 und/oder des Behälters 12 angeordnet sein können. Eine (ebenfalls nicht dargestellte) Sicherung, z.B. eine Feinsicherung oder eine Thermosicherung, schütz die Steuerung 60 und die Wärmemanschette 42 vor Überhitzung und dadurch gegebenenfalls auftretenden Beschädigungen.

Für eine individuelle Einstellung der Temperatur ist die elektrische Steuerung 60 mit einem Potentiometer 62 versehen, das innerhalb oder außerhalb des Notfallbehälters 20 zugänglich sein kann. Eine bevorzugt von außen gut sichtbare Kontrolleuchte 64 zeigt die aktive Verbindung der Wärmemanschette 42 mit der externen Stromversorgung 30 an, damit die Infusionsflaschen 12 bei Nichteinsatz stets vorgewärmt werden.

Als Stromversorgung 30 verwendet man zweckmäßig das Bordnetz eines (nicht gezeichneten) Rettungsfahrzeugs, beispielsweise eines Rettungswagens oder eines Rettungshubschraubers, wobei die elektrischen Stromkontakte 52 der Wärmemanschette 42 bzw. der Heizfolie 43 derart im Außenbereich des Notfallbehälters 20 angeordnet sind, daß durch bloßes Abstellen des Behälters 20 in einem dafür vorgesehenen Bereich oder an einem dafür vorgesehenen Platz in dem Rettungsfahrzeug eine elektrische Verbindung zwischen der Stromversorgung 30 und der Wärmemanschette 42 herstellbar ist.

Man kann die Heizvorrichtung 10 aber auch an ein hausinternes Stromnetz, an eine Batterie oder einen Akkumulator anschließen, der beispielsweise in dem Rettungsfahrzeug oder einer Rettungsstation vorhanden ist. Ein regeneratives Solarzellensystem kann als direkte Energieversorgung oder als Pufferung des Akkus oder der Batterie dienen.

Das Notfallbehältnis 20 kann gemäß Fig. 3 ein Notfallkoffer K sein, der in einem robusten aufklappbaren Gehäuse G mehrere Aufnahmefächer bzw. Fachunterteilungen U zur Aufnahme der für einen Notfall erforderlichen Utensilien aufweist. In einem separaten Aufnahmefach 22 sind zwei Wärmemanschetten 42 vorgesehen, die über ihre Zuleitungen 47 mit den Stromkontakten 52 verbunden sind. Letztere sind als Schleifkontakte mit relativ großen Kontaktflächen ausgebildet und in einer Seitenwand S des Koffers K derart eingelassen, daß sie von außen gut zugänglich sind. Die Zuleitungen 47 der Manschetten 42 können ebenfalls in der Seitenwand S des Koffers K eingelassen sein, ebenso die elektrische Steuerung 60, damit diese im Inneren des Koffers nicht stören.

In der Vorbereitung für einen Notfalleinsatz wird der Notfallkoffer K in bekannter Weise bestückt. In die Wärmemanschetten 42 wird jeweils eine Flasche 12 mit Infusionslösung eingelegt, wobei die Klettbänder 48 der Manschette 42 stets ein rasches und bequemes Auswechseln der Flasche 12 gewährleisten. Der Klettverschluß 48 ermöglicht ferner eine rasche Anpassung der Manschette 42 an allgemein gebräuchliche Formen von Infusionsbehältern 12, die auch unterschiedliche Formen und Abmessungen aufweisen können. Je nach Ausführungsform der Wärmemanschetten 42 können diese in dem Aufnahmefach 42 des Koffers K mittels weiterer (nicht gezeigter) Klettverbinder festgelegt werden, d.h. die Wärmemanschette 42 selbst kann als mobile Einheit dem Notfallkoffer K entnommen und unmittelbar am Notfallort eingesetzt werden. Die Wärmeisolierung 45 verhindert ein zu rasches abkühlen der Infusionslösung. Man kann die Manschette 42 aber auch mit einem erforderlichen Bewegungsspiel fest in dem Koffer K installieren. Die mechanisch stabile Ummantelung 46 hält in Verbindung mit der Wärmeisolierung 45 selbst stärkeren Belastungen problemlos stand.

Nach der Bestückung stellt man den Koffer K in ein im Rettungsfahrzeug vorgesehenes, den Abmessungen des Koffers K angepaßtes Kofferfach, wobei die Schleifkontakte 52 in der Seitenwand S des Koffers K zwangsweise mit geeigneten Gegenkontakten 53 der bordseitigen Stromversorgung 30 in Kontakt treten. Die Wärmefolie 43 der Wärmemanschette 42 beginnt unmittelbar zu heizen, so daß die in der Infusionsflasche 12 bevorratete Infusionslösung auf eine für den nächsten Einsatz erforderliche Temperatur gebracht und auf dieser, gegebenenfalls vorwählbaren Temperatur gehalten wird. Der Notfallkoffer K ist stets optimal mit allen erforderlichen Erste-Hilfe-Utensilien ausgestattet und kann ohne zusätzliche Maßnahmen unmittelbar aus dem Kofferfach entnommen werden. Die elektrischen Anschlüsse 50 und die Klettbänder 48 zum Festlegen der Manschette 42 an der Infusionsflasche 12 gewährleisten stets eine rasche und einfache Handhabung des Notfallkoffers K, so daß am eigentlichen Unfallort stets eine ausreichend temperierte Infusionslösung zur Hand ist. Fehlen in dem Notfallkoffer eine oder mehrere Infusionsbehälter, so kann dies über die Kontrolleuchte 64 angezeigt werden, wobei für jede Wärmemanschette 42 eine eigene Kontrolleuchte 64 vorgesehen sein kann.

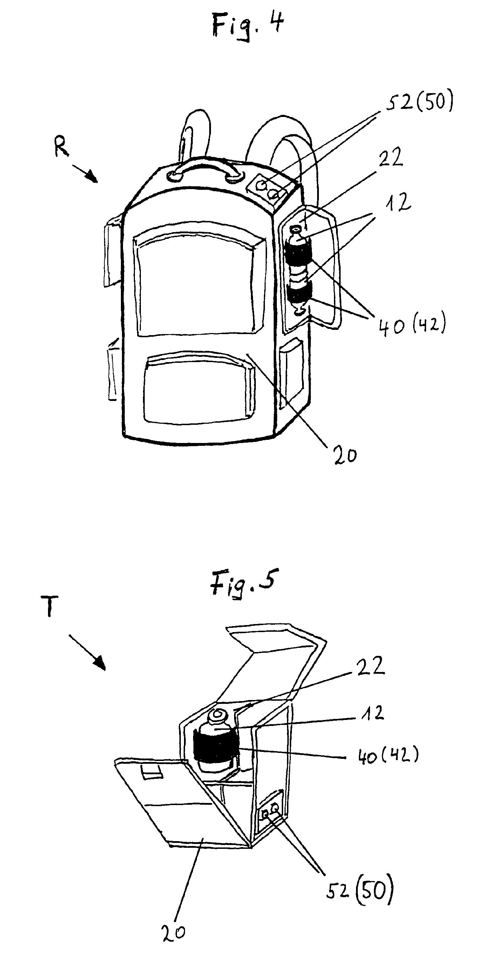

Eine andere Ausführungsform eines tragbaren Notfallbehälters 20 in Form eines Rucksacks R ist in Fig. 4 dargestellt. Die Wärmemanschetten 42 sind in einem seitlichen Aufnahmefach 42 untergebracht, während die elektrischen Anschlüsse 50 in Form von Magnetkontakten 52 an der Oberseite O des Rucksacks R ausgebildet sind. Stellt man den Rucksack R in einem Rettungsfahrzeug oder in einer Rettungsstation ab, wird die externe Stromversorgung 30 über Magnetkontakte 53 mit der Heizung 40 des Rucksacks R verbunden, indem man die Kontakte 52, 53 einfach zusammenführt. Unterschiedliche Gestaltungen der Kontakte 52, 53 sichert eine korrekte Polung der Energieversorgung. Im Einsatzfall entnimmt man den Rucksack R seiner Aufbewahrung, wobei sich die Kontakte 52, 53 selbsttätig lösen. Die in den Manschetten 42 untergebrachten Infusionsflaschen 12 wurden bis dahin permanent beheizt, so daß die darin bevorratete Infusionslösung eine für den menschlichen Körper verträgliche Temperatur aufweist und sofort eingesetzt werden kann. Der Rucksack R steht mit samt seinem Inhalt ohne jegliche Zeitverzögerung sofort zur Verfügung.

Fig. 5 zeigt eine noch andere Ausbildung der Erfindung in einer Notfalltasche T. Die Infusionsflasche 12 ist von einer Wärmemanschette 42 umschlossen in einem Aufnahmefach 22 untergebracht und über seitliche Kontakte 53 an eine externe Stromversorgung 30 anschließbar. Stellt man die Tasche T in einem vorgesehenen Fach oder an einem geeigneten Platz ab, treten die Stromanschlüsse 52 automatisch mit den Anschlüssen 53 einer separaten Stromversorgung 30 in Kontakt. Die Heizeinrichtung 40 wird unmittelbar mit Strom versorgt und beheizt die Infusionsflasche 12, während die Notfallausrüstung in den dafür jeweils vorgesehenen Fächern ruht. Nimmt man die Tasche T für einen Einsatz auf, wird die elektrische Kupplung 52, 53 selbsttätig gelöst, ohne daß weitere Handgriffe erforderlich sind.

Die Vorrichtung 10 zum Erwärmen und/oder Warmhalten von Infusionslösungen kann bereits werkseitig in den Notfallbehältnissen 20 integriert sein. Sie kann aber auch jederzeit problemlos in Notfallkoffern oder Rucksäcken nachgerüstet werden.

Die Erfindung ist nicht auf eine der vorbeschriebenen Ausführungsformen beschränkt, sondern in vielfältiger Weise abwandelbar. So kann man anstelle der die Infusionsflaschen 12 nur teilweise umschließenden Wärmemanschetten 42 sogenannte Wärmetaschen herstellen, welche die Infusionsflaschen oder -beutel 12 formschlüssig aufnehmen. Gefertigt werden die Wärmetaschen aus einem flexiblen und/oder textilen Material, in das eine Heizeinrichtung 40 bereits vollflächig integriert ist. Der Anschluß einer solchen Wärmetaschen an die Stromversorgung 30 erfolgt in der bereits beschriebenen Art und Weise.

Man kann ferner auf die elektrische Steuerung 60 ganz verzichten und Heizeinrichtungen 40 wählen mit fest vorgegebenen konstanten Heizleistungen. Diese sind derart zu bemessen, daß die zu erwärmenden Infusionsflaschen 12 eine ausreichende Endtemperatur erreichen, ohne daß es zu Überhitzungen kommen kann.

Darüber hinaus kann man die Heizeinrichtung 40 beispielsweise unmittelbar in dem Aufnahmefach 22 des Notfallbehälters 20 integrieren, beispielsweise in einer Wandung oder einer Trennfläche. In einem solchen Fall würde man den Infusionsbehälter 12 formschlüssig in das Aufnahmefach 22 einsetzen, so daß stets ein ausreichender Wärmekontakt gegeben ist.

Eine zusätzliche in dem Notfallbehälter 20 vorgesehene Stromversorgung kann den Einsatzbereich zusätzlich erweitern, was insbesondere bei Berg-, Seenot-, Luft- und alpinen Rettungsdiensten von Vorteil sein kann.

Man erkennt, daß eine Vorrichtung 10 zum Erwärmen und/oder Warmhalten von Infusionslösungen in tragbaren Notfallbehältern 20, wie Notfallkoffern, -rucksäcken, -taschen u.dgl., wenigstens eine an eine externe Stromversorgung 30 anschließbare Heizeinrichtung 40 für Infusionsbehälter 12 besitzt. Jede Heizeinrichtung besitzt 40 ist über Stromkontakte 50, beispielsweise Magnet-, Steck- oder Schleifkontakte, mit der externen Stromversorgung 30 verbindbar, wobei die Stromkontakte 30 frei zugänglich im Außenbereich des Notfallbehälters 20 angeordnet sind. Die Heizeinrichtung 40 ist bevorzugt eine in einem Aufnahmefach 22 des Notfallbehälters 20, in einer Wärmemanschette 42 oder in einer Wärmetasche integrierte Widerstandsheizung, deren Leistung mittels einer elektrischen oder elektronischen Steuerung 60 regelbar ist.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

### Bezugszeichenliste

- 10: Vorrichtung
- 12: Infusionsbehälter

- 20: Notfallbehälter
- 22: Aufnahmefach

- 30: externe Stromversorgung

- 40: Heizeinrichtung
- 42: Wärmemanschette
- 43: Heizfolie
- 44: Rückseite
- 45: Wärmeisolierung
- 46: Ummantelung
- 47: elektrische Zuleitung
- 48: Klettband

- 50: elektrische Anschlüsse
- 52, 53: Stromkontakte

- 60: Steuerung
- 62: Potentiometer
- 64: Kontrolleuchte

## Patentansprüche

1. Vorrichtung (10) zum Erwärmen und/oder Warmhalten von medizinischen Lösungen, insbesondere Infusionslösungen, Transfusionslösungen u.dgl., in tragbaren Notfallbehältern (20), wie Notfallkoffern, -rucksäcken, -taschen u.dgl., mit wenigstens einer an eine externe Stromversorgung (30) anschließbaren Heizeinrichtung (40) für Infusionsbehälter (12) und mit elektrischen Anschlüssen (50) für die Heizeinrichtungen (40), wobei die Anschlüsse (50) derart im Außenbereich des Notfallbehälters (20) angeordnet sind, daß durch Abstellen des Notfallbehälters (20) in einem dafür vorgesehenen Bereich oder an einem dafür vorgesehenen Platz eine elektrische Verbindung zwischen der externen Stromversorgung (30) und der bzw. den Heizvorrichtungen (40) herstellbar ist.

2. Vorrichtung nach Anspruch 1, **dadurch gekennzeichnet**, daß die elektrischen Anschlüsse (50) Stromkontakte (52, 53) sind, beispielsweise Magnet-, Steck- oder Schleifkontakte.

3. Vorrichtung nach Anspruch 1 oder 2, **dadurch gekennzeichnet**, daß die Heizeinrichtungen (40) einem gemeinsamen elektrischen Anschluß (50) zugeordnet sind.

4. Vorrichtung nach einem der Ansprüche 1 bis 3, **dadurch gekennzeichnet**, daß jede Heizeinrichtung (40) eine Widerstandsheizung ist.

5. Vorrichtung nach einem der Ansprüche 1 bis 4, **dadurch gekennzeichnet**, daß jede Heizeinrichtung (40) eine fest vorgegebene konstante Heizleistung aufweist.

6. Vorrichtung nach einem der Ansprüche 1 bis 4, **dadurch gekennzeichnet**, daß die Heizleistung jeder Heizeinrichtung (40) mittels einer elektrischen oder elektronischen Steuerung (60) individuell oder einheitlich regelbar ist.

7. Vorrichtung nach Anspruch 6, **dadurch gekennzeichnet**, daß die Steuerung (60) in dem Notfallbehälter (20) integriert oder an dem Notfallbehälter (20) angebracht ist.

8. Vorrichtung nach Anspruch 7, **dadurch gekennzeichnet**, daß der Steuerung (60) wenigstens ein Temperatur-Meßfühler zugeordnet ist.

9. Vorrichtung nach einem der Ansprüche 7 oder 8, **dadurch gekennzeichnet**, daß die Steuerung (60) und/oder jede Heizeinrichtung (40) eine Sicherung aufweist.

10. Vorrichtung nach einem der Ansprüche 1 bis 9, **dadurch gekennzeichnet**, daß die Heizeinrichtung (40) in einem Aufnahmefach (22) des Notfallbehälters (20) integriert ist.

11. Vorrichtung nach Anspruch 10, **dadurch gekennzeichnet**, daß in das Aufnahmefach (22) wenigstens ein Infusionsbehälter (12) im wesentlichen formschlüssig einsetzbar ist.

12. Vorrichtung nach einem der Ansprüche 1 bis 9, **dadurch gekennzeichnet**, daß die Heizeinrichtung (40) in einem zu einer Wärmetasche, einer Wärmehülle o.dgl. verarbeiteten, flexiblen und/oder textilen Material integriert ist, welches zumindest einen Infusionsbehälter (12) aufnimmt.

13. Vorrichtung nach einem der Ansprüche 1 bis 9, **dadurch gekennzeichnet**, daß die Heizeinrichtung (40) eine lösbar an oder um einen Infusionsbehälter (12) festlegbare Wärmemanschette (42) aufweist.

14. Vorrichtung nach Anspruch 12 oder 13, **dadurch gekennzeichnet**, daß die Wärmetasche bzw. die Wärmemanschette (42) zumindest abschnittsweise eine Heizfolie (43) aufweist und auf ihrer dem zu erwärmenden Infusionsbehälter (12) abgewandten Rückseite (44) mit einer Wärmeisolierung (45) versehen ist.

15. Vorrichtung nach Anspruch 14, **dadurch gekennzeichnet**, daß die Wärmeisolierung (45) aus einem elastischen, formstabilen Material besteht und daß die Heizfolie (43) und die Wärmeisolierung (45) von einer mechanisch belastbaren Ummantelung (46) eingeschlossen sind.

16. Vorrichtung nach einem der Ansprüche 12 bis 16, **dadurch gekennzeichnet**, daß die Wärmetasche bzw. die Wärmemanschette (42) den bzw. die Infusionsbehälter (12) im wesentlichen formschlüssig umschließt und mittels Klettbändern (48), Riemenbändern, einer Gummimanschette o.dgl. verschließbar ist.

17. Vorrichtung nach einem der Ansprüche 12 bis 17, **dadurch gekennzeichnet**, daß die Wärmetasche bzw. die Wärmemanschette (42) in dem Notfallbehälter (20) festlegbar ist.

18. Vorrichtung nach einem der Ansprüche 12 bis 18, **dadurch gekennzeichnet**, daß die Wärmetasche bzw. die Wärmemanschette (42) in dem Notfallbehälter (12) fest installiert ist.

19. Vorrichtung nach einem der Ansprüche 1 bis 18, **dadurch gekennzeichnet**, daß eine zusätzliche Stromversorgung in dem Notfallbehälter vorgesehen ist, beispielsweise eine Batterie, ein Akkumulator, eine Solarzelle o.dgl.

20. Notfallbehälter (20), wie Notfallkoffer, -rucksack, -tasche u.dgl., mit einer Vorrichtung (10) nach einem der Ansprüche 1 bis 19.

21. Rettungsfahrzeug oder -flugzeug mit einer Einrichtung zur Aufnahme und/oder Bereithaltung eines Notfallbehälters (20) nach Anspruch 20.
